# EUROPEAN PATENT APPLICATION

(11) **EP 2 108 345 A1**
(43) Date of publication of application: **14.10.2009**
(21) Application number: 08075293.4
(22) Date of filing: 11.04.2008
(51) Int. Cl.: A61F 5/445, A61F 5/448

(54) **A 1-piece stoma system with a soft, extensible stoma opening**

(71) Applicant: Eurotec Beheer B.V., 4705 AG Roosendaal (NL)
(72) Inventor: Van der Leden, Arie Gijsbert, 2920 Kalmthout (BE)

(57) **Abstract**

A 1-piece stoma (ostomy)* system, comprising of a stoma (ostomy*) pouch which is welded on a skin friendly adhesive skin barrier, which is laminated with a soft and flexible polymeric foam layer. In this invention the pouch is not welded directly around the stoma opening of the skin barrier, but is welded along the circumference of a relatively large die cut opening in the back side pouch film(s), thus keeping the direct peri-stomal of the skin barrier free from pouch film, thus allowing the laminated adhesive foam layer to be adjusted in size by stretching the opening manually or by expansion of the stoma itself, for instance due to the passage of voluminous solid stool or due to swelling of the stoma in the post operative period. The soft and extensible peri-stomal area also allows the laminated adhesive to follow the contours of the skin and even adhere to uneven or retracted skin areas.

## Description

### Background of the invention

A number of diseases involving the intestinal and urinary tracts may result in the construction of an artificial outlet on the abdominal area for stool and/or urine.

In the case of diversion of the large intestine into the abdominal wall, one talks about a colostomy. In the case of diversion of the small intestine one talks about an ileostomy. Thirdly in the case of an urostomy, the urine is disposed through an artificial opening in the abdominal wall.

The artificial openings of a colostomy, ileostomy and a urostomy are collectively referred to as stomas. During the past years a large variety of stoma appliances have been developed. Patients with a colostomy collect their relatively solid faeces in closed pouches, which usually are changed several times a day. Patients with an ileostomy collect their relatively liquid stool in drainable pouches, the opening of which can be opened and closed with a tail clip. Patients with a urostomy collect their urine in a pouch with a drain tap. Ileostomy and urostomy pouches are usually changed once a day.

Stoma appliances are to be divided in two main systems; the 1-piece and the 2-piece system. The design of most 2-piece stoma systems is based on the 2-piece stoma system designed by Steer et al. (British patent application 571, 657). This stoma system consists of a body side wafer produced from a special hydrocolloid adhesive, covered by a thin polyethylene film, on which a circular shaped polymeric flange with an upstanding or projecting rib has been attached , which functions as a coupling member. The stoma pouch is also provided with a circular shaped coupling member, which can be attached on or around the projecting rib of the body side flange.

In the case of a 1-piece system, the skin barrier - which consists of a special adhesive - is integral with the pouch. This type of pouch is designed to be affixed directly to the skin, and has the advantage that the total surface of the adhesive is flatter and more flexible, which allows it more or less to follow the contours of the skin. A major disadvantage of most current 1-piece and 2-piece systems is the rigidity of the stoma opening. Most 2-piece body side wafers are laminated with a rigid polymeric - usually polyethylene - film to obtain a reliable weld with the polymeric coupling member. This film however is not only rigid, but also not stretchy, so that the stoma opening cannot expand when solid stool is passing or when the stoma is swelling in the post-operative period due to oedema.

This may result in stool undermining the wafer and causing leakage or even result in damaging the mucus of the stoma when the rigid stoma opening strangles the expanding stoma. Moreover, a body side wafer or skin barrier, laminated with such a rigid polymeric film, bears a major potential risk of cutting the stoma, in the case its adhesive dissolves due to aggressive stoma output, whereby the sharp edges of the remaining polymeric film may act as a razor blade. The inventor of this invention has earlier solved this problem by replacing the polyethylene film of a 2-piece wafer by a polyethylene foam layer in combination with a special ultrasonic welding technique. (EU patent application 02080205.4-1526; US patent application 10/866,798). When producing 1-piece pouches, one usually also uses adhesive skin barriers laminated with a polymeric film, or alternatively with a non-woven top layer. Although non-woven layers are of an extremely soft material, the extensibility of an adhesive skin barrier laminated with a non-woven is almost zero, so that one faces identical problems with 1- piece pouches in the case the stoma diameter will expand. This also applies for skin barriers of 1-piece pouches, which are laminated with a polymeric film, Moreover, even in the case one should laminate the 1-piece skin barrier with a stretchy material, like a polymeric foam layer, the flexibility and extensibility of the stoma opening in the skin barrier will be nullified by the pouch film, which usually is welded over a circular area of the skin barrier starting directly around the stoma opening.

### Description of the invention

In this invention the adherent 1-piece skin barrier is provided with a polymeric foam layer, which is kept plain and free from any welded material in its peri-stomal area. This is achieved by die cutting an ample aperture in the pouch film to be welded, which aperture is kept significant larger in diameter than the pre-cut or cut to size stoma-opening of the 1-piece pouch. In this invention the die cut opening in the pouch film is 45 mm for stomas up to 35 mm and 70 mm for stomas up to 60 mm in diameter, but any other opening will be suitable as long as it will be substantial larger in diameter than the diameter of the stoma. So in contrast with other current and common 1-piece pouches, the back side pouch film in this invention is only welded along the circumference of its die cut opening with a weld of say 12-15 mm wide, keeping the direct peri-stomal area of the skin barrier inside the pouch free from welded pouch film, but other weld widths will do as well.

The adhesive layer of the body side wafer - in this invention consisting of hydrocolloid, is covered by a soft stretchy polymeric foam layer, allowing the stoma opening to be stretched manually or by expansion of the stoma itself. This will enable the stoma to dispose of voluminous solid stool without undermining the adhesive layer or any risk of damaging the mucus of the stoma. Also in the case of post-surgical swelling of the stoma, for instance due to oedema, the stoma-opening in the free polymeric foam layer, will easily follow the increasing circumference of the stoma. It will also allow the user to stretch the stoma opening a little bit in case the pre-cut opening should be somewhat too narrow. This means that - if these 1-piece pouches are offered with a variety of pre-cut openings, with steps of say 3 mm in diameter - in most cases patients do need to cut any in-between stoma-opening, as they can easily use the one step smaller standard opening en stretch it to size. Another advantage of such a flexible and extensible peri-stomal area that the adhesive can easily follow the contours of the skin and even adhere to uneven or retracted skin areas.

### Description of the drawings

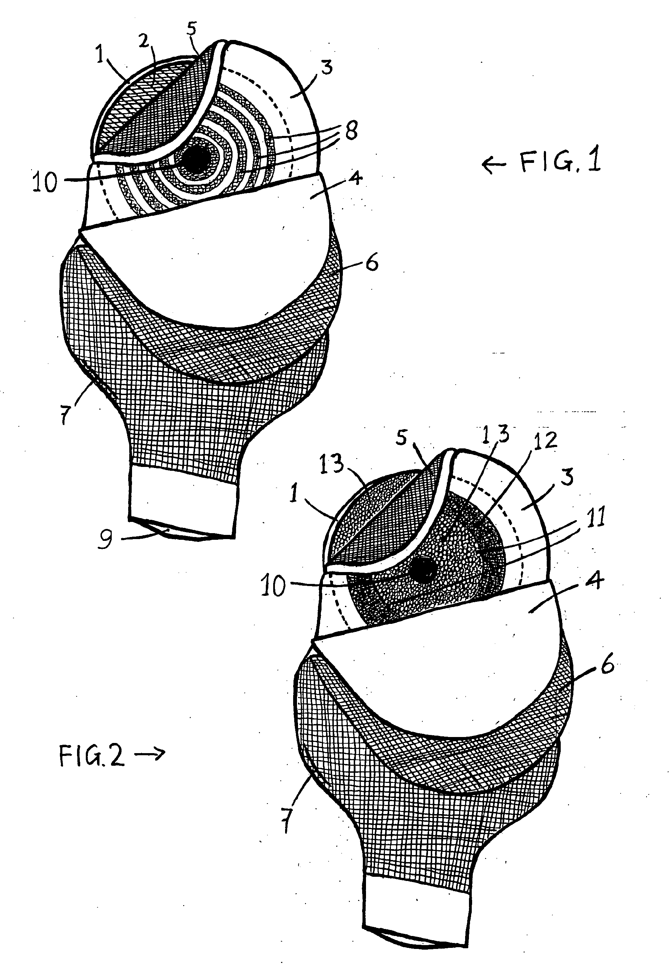

Fig. 1 shows the composition of a very common and traditional 1-piece stoma pouch; in this case a drainable (ileostomy) pouch, but alternatively it could easily be another type of pouch, such as a closed (colostomy) pouch or a urostomy pouch, provided with a drainable bottom tap. It shows the adhesive layer (1) laminated with a weldable top layer in this case a layer of polymeric foam (2). The actual pouch is formed by two layers of plastic film (3 and 4) which on both sides are covered by a comfort layer of a breathing fabric (5 and 6), to increase the appearance and wearing comfort of the pouch. These 4 layers of film and fabric are welded together over the full circumference of their outer mutual edges (7), except for of course the bottom outlet of the drainable pouch (9). The pouch film (3) and comfort layer (5) are welded to the laminated adhesive layer (1 and 2) by several intermittent circular welds (8), but alternatively it could also easily be done by means of one wide continuous weld. Usually the comfort layer of a breathing fabric (5) and the pouch film (3) are both welded together to the laminated adhesive (1 and 2), but sometimes the fabric (5) is provided with an aperture around the welding area of the adhesive to keep it free from the peri-stomal welding area of the pouch film (3). This may be done for maintaining an access to a possible carbon filter, welded on pouch film (3). It may also be done in the case the comfort layer (5) cannot be welded on the laminated surface of the adhesive layer (2). The welded combination of laminated adhesive (1 en 2) and the pouch film (3) possibly together with comfort film (5) are provided with a pre cut stoma opening or a start opening to be cut to size by the user (10).

Fig. 2 shows the composition of a rather similar 1-piece stoma pouch; in this case also a drainable (ileostomy) pouch, but alternatively it could be another type of pouch as well, such as a closed (colostomy) pouch or a urostomy pouch provided with a drainable tap. It also shows the adhesive layer (1) laminated with a weldable top layer (2). Also here the actual pouch is formed by two layers of plastic film (3 and 4) which on both sides are covered by a comfort layer of a breathing fabric (5 and 6), to increase the appearance and wearing comfort of the pouch. These 4 layers of film and fabric are welded together over the full circumference of their outer mutual edges (7), except for of course the bottom outlet of the drainable pouch (9). The difference with Fig. 1 - which in fact is the quintessence of this invention, is that the pouch film (3) and the comfort layer (5) are provided with an aperture, which aperture is significant larger in diameter than the pre-cut or cut to size stoma-opening (10) of the 1-piece pouch. In this invention the die cut opening in the pouch film (3) is 45 mm for stomas up to 35 mm and 70 mm for stomas up to 60 mm in diameter, but any other opening will be suitable as long as it will be substantial larger in diameter than the diameter of the stoma. So in contrast with the pouch in Fig 1 and other current and common 1-piece pouches, the pouch film (3) and the comfort layer (5) of this invention are not welded on the direct peri-stomal area of skin barrier, but only welded along the circumference of its ample die cut opening (11) with an intermittent or continuous weld (12), thus keeping the direct peri-stomal area inside the pouch consisting of a with polymeric foam laminated adhesive skin barrier (13), free from any welded pouch film. Alternatively, only pouch film (3) is welded around its aperture (11) to the with polymeric foam laminated adhesive skin barrier (13), whereby the fabric comfort layer (5) is provided with an aperture around the welding area (12), so that it will allow access to a possible carbon filter, welded on pouch film (3). This may also be the case, if comfort layer (5) is technically unable to be welded on the laminated surface of the laminated skin barrier (13).

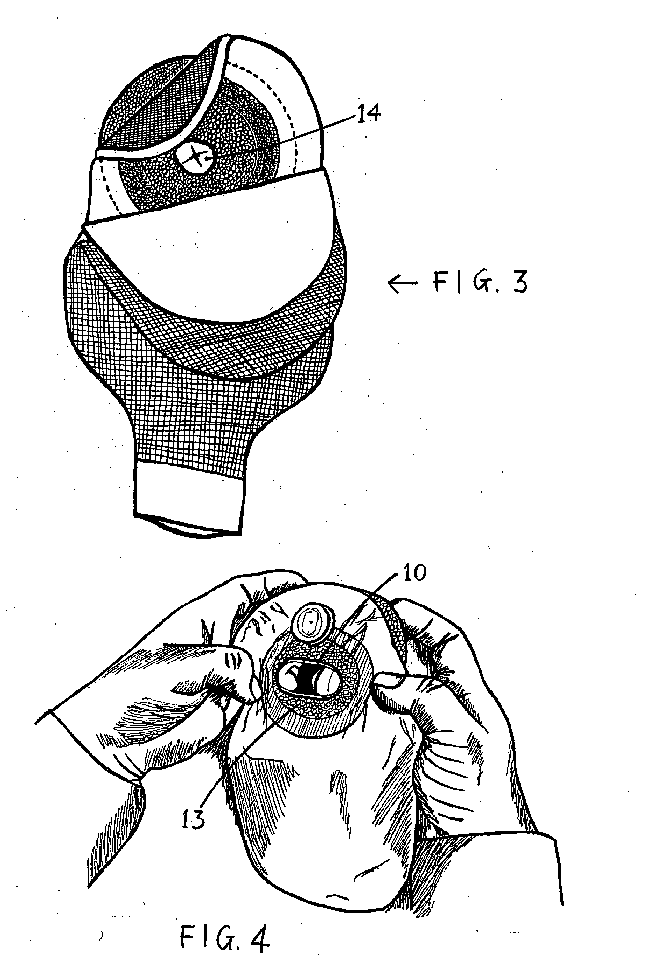

Fig. 3 shows the 1-piece pouch of Fig. 2, whereby the stoma opening is attached around a stoma.

Fig. 4 shows the advantages of the invention, in this case demonstrated with a transparent closed (colostomy) pouch, showing the flexibility and particularly the extensibility of the stoma opening, as a result of keeping the peri-stomal area of the with polymeric foam laminated skin barrier (13) free from welded pouch film(s).

## Claims

1. A 1-piece stoma pouch, including an integral skin barrier, which skin barrier is laminated with a polymeric foam layer, and of which the skin facing pouch film is welded on said skin barrier along the circumference of an ample die cut aperture in said skin facing pouch film, which die cut aperture is considerably larger in diameter than the starter opening or pre-cut stoma opening in said skin barrier, thus keeping the peri-stomal area of said skin barrier free from any welded pouch film, so that the said skin barrier in the peri-stomal area is not limited in its flexibility and extensibility.

2. A 1-piece stoma pouch, according to claim 1, including an integral skin barrier, which skin barrier is laminated with a polymeric foam layer, and of which the skin facing pouch film together with a breathing comfort layer, are welded on said skin barrier along the circumference of an ample die cut aperture in said combination of skin facing pouch film and breathing comfort layer, which die cut aperture is considerably larger in diameter than the starter opening or pre-cut stoma opening in said skin barrier, thus keeping the peri-stomal area of said skin barrier free from any welded pouch film, so that the said skin barrier in the peri-stomal area is not limited in its flexibility and extensibility.

3. A 1-piece stoma pouch, including an integral skin barrier, which skin barrier is laminated with a polymeric foam layer, and of which the skin facing pouch film is bonded on said skin barrier along the circumference of an ample die cut aperture in said skin facing pouch film, which die cut aperture is considerably larger in diameter than the starter opening or pre-cut stoma opening in said skin barrier, thus keeping the peri-stomal area of said skin barrier free from any welded pouch film, so that the said skin barrier in the peri-stomal area is not limited in its flexibility and extensibility.
